Europäisches Patentamt

(19) European Patent Office

Office européen des brevets

(11) Publication number: **0 139 351**
**B1**

# (12) EUROPEAN PATENT SPECIFICATION

(45) Date of publication of patent specification: **17.05.89**

(21) Application number: **84304833.1**

(22) Date of filing: **16.07.84**

(51) Int. Cl.⁴: **A 61 F 5/44,** A 61 F 5/48

(54) Incontinence insert.

(30) Priority: **18.07.83 US 514620**

(43) Date of publication of application:
**02.05.85 Bulletin 85/18**

(45) Publication of the grant of the patent:
**17.05.89 Bulletin 89/20**

(84) Designated Contracting States:
**AT BE CH DE FR GB IT LI LU NL SE**

(56) References cited:
**EP-A-0 062 495**

(73) Proprietor: **E.R. Squibb & Sons, Inc.**
**Lawrenceville-Princeton Road**
**Princeton, N.J. 08540 (US)**

(72) Inventor: **Gegelys, Anthony A.**
**133 Drake Road**
**Somerset New Jersey (US)**

(74) Representative: **Thomas, Roger Tamlyn et al**
**D. Young & Co. 10 Staple Inn**
**London WC1V 7RD (GB)**

Courier Press, Leamington Spa, England.

## Description

The present invention relates to incontinence devices and, more particularly, to an incontinence insert designed for use with conventional undergarments in order to contain both liquid and solid waste material.

Many individuals, either due to advanced age or illness, are unable to control or restrain the natural discharge of waste material. Individuals who are incontinent with respect to liquid waste normally utilize an incontinent garment in the form of a brief or the like which has a pocket therein. Into the pocket is inserted a disposable moisture-absorbent pad which can be changed as necessary. The garment itself is normally washable, such that it can be used repeatedly. Individuals who are incontinent with respect to solid waste material normally have to utilize an adult version of a diaper which is fitted to their body contours and will contain excreted solid waste material.

In both cases, the garment or diaper may be a source of embarrassment to the adult individual because of the stigma involved in wearing such garments. Thus, while the use of such garments is intended to permit the incontinent individual to lead as normal a life as possible, the wearing of an incontinent garment or diaper may itself restrict some of the activities in which the individual would like to participate.

In addition, with respect to both incontinence garments which accept a removable pad and adult diapers, it is necessary that the garment or diaper be correctly fitted to the contours of the body of the individual. In particular, it is necessary that the garment or diaper fit snugly around the waist and hips of the wearer in order to look and function properly. Accordingly, such garments and diapers have been provided, either in individual sizes to suit various sized individuals, or in a single size which is adjustable by means of elastic waistbands, snaps, or size adjusting adhesive tabs or the like to permit the individual the necessary adjustability.

While there are incontinence garments fabricated for use by individuals who cannot control liquid waste and other types of incontinence garments designed for use by individuals who cannot control solid waste, there are few garments which are comfortable and easy to change. The garments designed for use to absorb liquid waste are normally highly moisture-absorbent, but are incapable of effectively encapsulating solid waste.

As background prior art, it should be mentioned that European Patent Application EP—A—62495 describes an incontinence pad of absorbent sheet material which may be adhered to conventional fabric underpants. The document also discloses the possibility of removably housing the pad in a sheath which may be of a non-woven fabric made from polyester and which may be provided with a water-impervious sheet on its outer side (i.e. the side away from the wearer). The absorbent sheet material may be a multi-layer material, at least one layer of which is provided with openings. Composites including layers of tissue are described and modified starches can be used as the absorbent material.

In accordance with the present invention, there is provided an insert for use by incontinent individuals in a conventional undergarment, the insert comprising a substantially hourglass-shaped body with cut-out sections to accommodate the legs of the individual, said body comprising an inner (i.e. adjacent the individual's skin when in use) layer of non-woven moisture permeable polyester or rayon fiber, an outer layer of moisture impermeable polyethylene film, and a layer of moisture absorbent fluffed wood pulp core material, said inner layer and said outer layer being joined along the periphery of said body to enclose said absorbent core material, a centrally located portion situated between said cut-out sections and interposed between said inner layer and said absorbent material, said portion comprising a uni-directional barrier layer of substantially uniform thickness and a moisture permeable wicking layer of tissue material situated between said barrier layer and said inner layer, said barrier layer comprising a laminate containing a polymer that expands when wetted, said barrier layer having a plurality of apertures therethrough, said apertures having a given diameter prior to the expansion of said polymer to facilitate the passage of liquid through said barrier layer to said absorbent core material, the diameter of said apertures decreasing as said polymer expands so as to restrict the passage of liquid back through said barrier layer towards said permeable inner layer.

Adhesive means may be mounted on the outer layer to releasably secure the insert to an undergarment.

The uni-directional barrier layer is preferably a laminate of a "super absorbent" polymer and a base or carrier of paper or the like. Suitable commercially available "super absorbent" polymers include starches, acrylics, modified cellulose, gums and the like. The polymer material may be coated onto a paper base or sandwiched between layers of paper to form the laminate. The "super absorbent" polymer, once it becomes wet, will gel and expand. The barrier layer also includes a number of apertures extending through the barrier layer and spaced across the surface of the barrier layer. These apertures facilitate the movement of moisture from the moisture permeable inner layer, through the barrier layer, and into the moisture absorbent core material. As the polymer becomes wet and gels, it expands, thereby reducing the size of the apertures and preventing moisture from moving back through the barrier layer from the core material to the permeable inner layer which is in contact with the skin of the wearer. Thus, the apertured layer acts to retain moisture in the absorbent core material by functioning as a uni-directional moisture barrier.

The adhesive means normally comprises first and second adhesive strips situated proximate the ends of the body of the inert. These strips permit the insert to be snugly anchored within the undergarment.

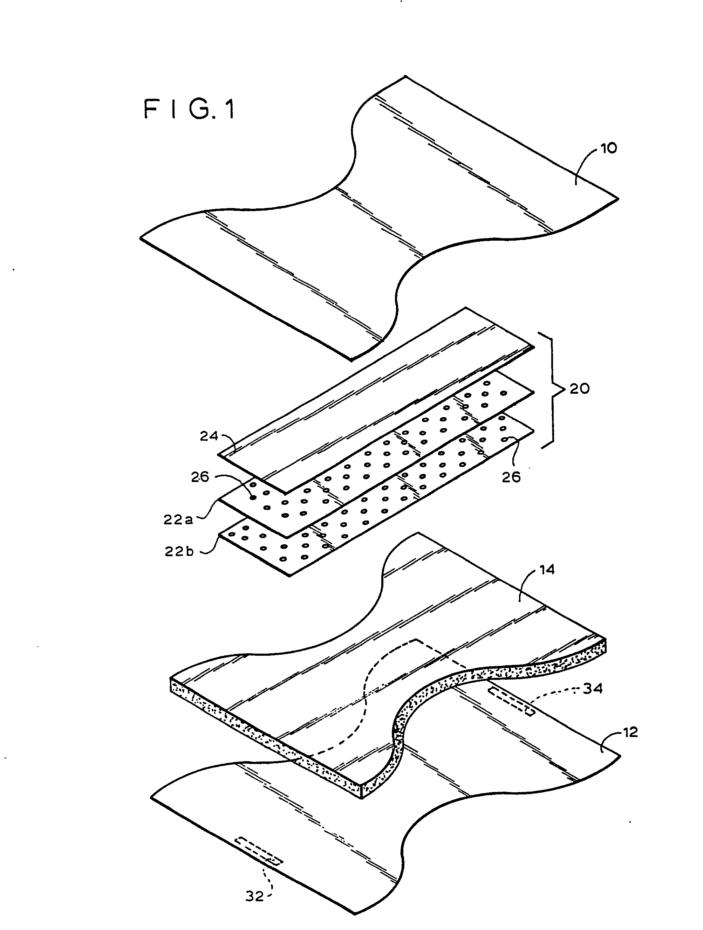

Fig. 1 is an exploded isometric view of a preferred incontinence insert of the present invention;

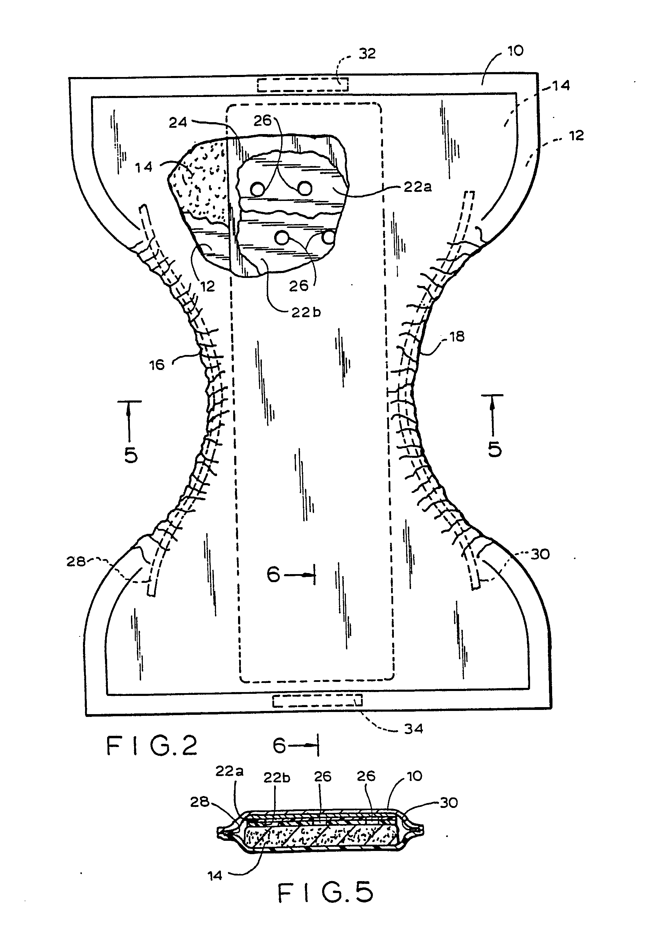

Fig. 2 is a top plan view of the incontinence insert of Fig. 1;

Fig. 3 is a front view of the incontinence insert of Fig. 1 shown folded;

Fig. 4 is a side view of the incontinence insert of Fig. 1 shown folded;

Fig. 5 is a cross-sectional view taken along line 5—5 of Fig. 2; and

Fig. 6 is a cross-sectional view taken along line 6—6 of Fig. 2.

As shown in the drawings, the preferred incontinence insert of the present invention has a substantially hourglass-shaped body formed of an hourglass-shaped inner layer 10 composed of a non-woven, moisture-permeable polyester or rayon fibers, which permit the passage of fluid therethrough. Inner layer 10 is attached to a similar shaped outer layer 12 to enclose the remaining portions of the insert. Layer 12 is composed of a moisture impermeable polyethylene film. The peripheries of inner layer 10 and outer layer 12 are affixed to each other by adhesive or the like so as to form an hourglass-shaped interior recess.

The recess is filled with a layer of moisture absorbent core material 14 that also has an hourglass shape, but is of dimensions slightly smaller than the dimensions of inner layer 10 or outer layer 12. The moisture absorbent core material is composed of fluffed wood pulp.

Situated between the semicircular cut-out portions 16 and 18 designed to accommodate the legs of the wearer, and extending along the vertical center line of the insert, from a point proximate one end thereof to a point proximate the other end thereof, is a generally rectangular portion 20. Portion 20 is formed of a uni-directional barrier layer 22 and a wicking layer 24. Layers 22 and 24 are situated between the moisture absorbent core material 14 and the interior surface of inner layer 10 with layer 22 adjacent the moisture core absorbent material 14 and layer 24 adjacent the interior surface of inner layer 10. Barrier layer 22 functions to retain liquid within material 14.

Uni-directional barrier layer 22 is of a planar, rectangular shape and is of substantially uniform thickness. The "super absorbent" polymer constituent of barrier layer 22 gels and expands when wet. Thus, this layer will act to block the transfer of moisture therethrough. Barrier 22, if formed of an uninterrupted laminate, would gradually restrict the amount of moisture passing therethrough and, finally, after being completely gelled and expanded, would effectively block any moisture from passing into moisture absorbent material 14.

To enhance the amount of fluid, i.e., urine, which can pass through uni-directional barrier layer 22, barrier layer 22 is provided with a large number of apertures 26. The shape, size, and number of apertures can be varied according to the gelling and expansion characteristics of the particular "super absorbent" polymer employed. As layer 22 gradually undergoes the transition which results in gelling and expanding, the swelling of the "super absorbent" polymer substantially decreases the size of the apertures. Thus, the passage of moisture through the barrier layer is gradually reduced, but much more gradually than if no apertures were present.

Accordingly, barrier 22 will act to retain moisture within the absorbent core material by permitting a substantial amount of moisture to pass through it to absorbent core layer 14, but, thereafter, preventing any substantial amount of liquid from passing from absorbent core layer 14 back through barrier layer 22 and wicking layer 24 to the moisture permeable inner layer 10 and, thus, back to the wearer. The result is that the top of the insert will be more comfortable to the wearer because it will not have a wet feel.

Uni-directional barrier layer 22 may be a single sheet of material, several sheets arranged so that the apertures 26 are aligned as note sheets 22a and 22b in the figures, or a single sheet bent or folded in such a manner that apertures 26 are aligned. Preferred materials for barrier layer 22 are a laminate containing a "super absorbent" starch polymer known as DWAL (trademark of Dow Chemical) and a laminate containing a "super absorbent" modified acrylic polymer known as Gelok 4000 (trademark of Gelok International).

Wicking layer 24 is of a moisture permeable tissue paper material and it functions to more uniformly disperse the fluid, i.e., urine, passing through permeable inner layer 10 onto uni-directional barrier layer 22. Thus, wicking layer 24 assures that the "super absorbent" polymer component of barrier layer 22 will gel and expand more uniformly across the entire surface of layer 22.

The insert of the present invention is designed to be situated within a conventional undergarment. Cut-out portions 16 and 18 are each provided with an internal elastic strip 28, 30 which tends to gather the sections of inner layer 10 and outer layer 12 along the cut-out portions 16 and 18, such that the insert will fit snugly around the inside of the legs of the wearer.

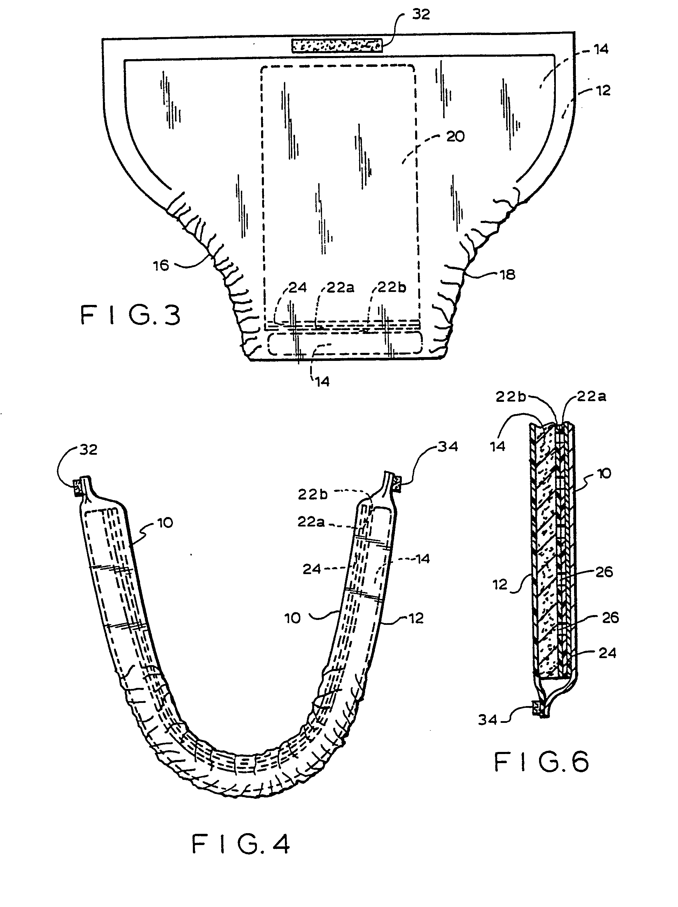

The exterior surface of outer layer 12 is provided with adhesive strips 32, 34 located proximate the edges of the body portion of the insert, as illustrated in Figs. 3 and 4, to permit the insert to be securely anchored to the interior of the undergarment. Strips 32, 34 will prevent the insert from moving around within the undergarment and enhance the comfort thereof.

It will now be appreciated that the present invention is usable with conventional undergarments, and thereby eliminates the necessity of the use of an incontinence garment and pad combination, or an adult diaper and, accordingly, the stigma attached thereto. In addition, one size of insert fits all, thereby eliminating the necessity for different size garments to fit the hips and waist of the wearer.

The insert has a relatively large surface area and, therefore, is capable of effectively encapsulating solid waste material. In addition, the central portion of the insert is provided with a uni-directional moisture barrier such that moisture absorbent material can absorb a large amount of liquid without leaking back to the skin of the wearer, thereby creating an uncomfortable wet feeling.

It will now be appreciated that the structure of the insert of the present invention permits it to be used effectively to both absorb a large amount of liquid waste and, at the same time, has a large enough surface area to effectively encapsulate solid waste material. Thus, a single insert can be used for both purposes.

A preferred incontinence insert according to this invention is as follows. Liquid permeable inner layer 10 is a non-woven polyester or rayon material of from 0.25 to 2 ounces per square yard (8.5 to 68 g/m²), most preferably at about 0.5 ounces per square yard (17 g/m²). Liquid impermeable outer layer 12 is a polyethylene film of from 0.5 mils (12.7 μm) to 3 mils (76.2 μm), most preferably at about 1 mil (25 μm) thickness. Wickling layer 24 is tissue material at from 1 to 3 mils (25.4 to 76.2 μm), most preferably about 2 mils (50.8 μm) thick. Uni-directional barrier layer or layers 22 are preferably of sufficient thickness and include an amount of "super absorbent" polymer so as to absorb from 30 to 100 times their weight in urine. Apertures 26 are preferably dimensioned so as to occupy from 15% to 35% of the surface area, most preferably about 25% of the surface area, of barrier layer or layers 22 prior to contact with moisture. Moisture absorbent core material 14 is preferably a fluffed wood pulp of 0.15 inches (3.8 mm) to 1.5 inches (38 mm) thick, most preferably 0.25 inches (6.4 mm) to 0.5 inches (12.7 mm).

It will be seen that the preferred insert:

(a) need not be sized to accommodate the contours of the wearer's body;

(b) is highly effective in absorbing liquid waste, as well as encapsulating solid waste;

(c) includes a uni-directional moisture barrier which permits liquid waste to be absorbed, but thereafter, prevents same from moving back towards the wearer, thereby preventing the insert from having an uncomfortable wet feel;

(d) can be fabricated using standard manufacturing techniques; and

(e) is composed of relatively inexpensive parts which function together reliably.

## Claims

1. An insert for use by incontinent individuals in a conventional undergarment, the insert comprising a substantially hourglass-shaped body with cut-out sections (16, 18) to accommodate the legs of the individual, said body comprising an inner (i.e. adjacent the individual's skin when in use) layer (10) of non-woven moisture permeable polyester or rayon fiber, an outer layer (12) of moisture impermeable polyethylene film, and a layer (14) of moisture absorbent fluffed wood pulp core material, said inner layer (10) and said outer layer (12) being joined along the periphery of said body to enclose said absorbent core material (14), a centrally located portion (20) situated between said cut-out sections (16, 18) and interposed between said inner layer (10) and said absorbent material (14), said portion (20) comprising a uni-directional barrier layer (22) of substantially uniform thickness and a moisture permeable wicking layer (24) of tissue material situated between said barrier layer (22) and said inner layer (10), said barrier layer (22) comprising a laminate containing a polymer that expands when wetted, said barrier layer (22) having a plurality of apertures (26) therethrough, said apertures (26) having a given diameter prior to the expansion of said polymer to facilitate the passage of liquid through said barrier layer (22) to said absorbent core material (14), the diameter of said apertures (26) decreasing as said polymer expands so as to restrict the passage of liquid back through said barrier layer (22) towards said permeable inner layer (10).

2. The insert of claim 1, further comprising adhesive means (32, 34) mounted on the outer layer (12) for releasably securing said insert to an undergarment.

3. The insert of claim 1, wherein said non-woven moisture permeable polyester or rayon fiber is of from 0.25 to 2 ounces per square yard (8.5 to 68 g/m²), said moisture impermeable polyethylene film is from 0.5 mils (12.7 μm) to 3 mils (76.2 μm), said tissue wicking layer (24) is from 1 to 3 mils (25.4 μm to 76.2 μm), said moisture absorbent fluffed wood pulp core (14) is from 0.15 inches (3.8 mm) to 1.5 inches (38 mm), and said apertures (26) constitute from 15% to 35% of the surface area of said uni-directional barrier layer (22) prior to contact with moisture.

4. The insert of claim 3, wherein said barrier layer (22) is a plurality of apertured sheets (22a, 22b), said apertures (26) being substantially aligned, each sheet being of substantially uniform thickness and comprising a laminate of a base or carrier and a polymer that expands when wetted.

5. The insert of claim 4, wherein said polymer is a starch polymer.

6. The insert of claim 4, wherein said polymer is an acrylic polymer.

7. The insert of claim 3, wherein said barrier layer (22) is an apertured sheet of substantially uniform thickness folded one or more times such that the apertures (26) are substantially aligned, said sheet comprising a laminate of a base or carrier and a polymer that expands when wetted.

8. The insert of claim 7, wherein said polymer is a starch polymer.

9. The insert of claim 7, wherein said polymer is an acrylic polymer.

10. The insert of any preceding claim, wherein there is a single layer of said absorbent core material (14).

## Patentansprüche

1. Einlage zur Verwendung durch inkontinente Personen in üblicher Unterwäsche, wobei die Einlage aufweist einen im wesentlichen sanduhrförmigen Körper mit Ausschnitten (16, 18) zur Aufnahme der Beine der Person, wobei der Körper eine Innenlage (10) (d.h. die im Gebrauch zur Haut der Person benachbart ist) aus nicht gewebten, feuchtigkeitsdurchlässigen Polyester- oder Kunstseidenfasern, eine Außenlage (12) aus feuchtigkeitsundurchlässigem Polyäthylenfilm und eine Lage (14) aus feuchtigkeitsabsorbierenden, geflockten Holzzellstoffkernmaterial aufweist, wobei die Innenlage (10) und die Außenlage (12) entlang des Rands des Körpers verbunden sind zum Einschließen des absorbierenden Kernmaterials (14), ein mittig angeordneter Abschnitt (20), der sich zwischen den Ausschnitten (16, 18) und zwischen der Innenlage (10) und dem absorbierenden Material (14) befindet, wobei der Abschnitt (20) eine Einweg-Barrierelage (22) mit im wesentlichen einheitlicher Dicke und eine feuchtigkeitsdurchlässige Flechtlage (24) aus feinem Gewebematerial, das zwischen der Barrierelage (22) und der Innenlage (10) angeordnet ist, aufweist, wobei die Barierelage (22) ein Laminat aufweist, das ein sich bei Nässe ausdehnendes Polymer enthält, wobei die Barrierelage (22) mehrere durchgehende Öffnungen (26) hat, wobei die Öffnungen (26) vor der Ausdehnung des Polymers einen vorgegebenen Durchmesser haben, wodurch der Durchgang von Flüssigkeit durch die Barrierelage (22) zu dem absorbierenden Kernmaterial (14) erleichtert wird, wobei der Durchmesser der Öffnungen (26) abnimmt, wenn sich das Polymer ausdehnt, so daß der Rückfluß von Flüssigkeit durch die Barrierelage (22) zu der durchlässigen Innenlage (10) begrenzt wird.

2. Einlage nach Anspruch 1, ferner mit Klebeeinrichtungen (32, 34), die auf der Außenlage (12) befestigt sind zum lösbaren Sichern der Einlage in der Unterwäsche.

3. Einlage nach Anspruch 1, wobei die nicht gewebten feuchtigkeitsdurchlässigen Polyester- oder Kunstseidenfasern von 0,25 bis 2 Unzen pro Square Yard (8,5 bis 68 g/m²), der feuchtigkeitsundurchlässige Polyäthylenfilm von 0,5 mils (12,7 µm) bis 3 mils (76,2 µm) die Feingewebeflechtlage (24) von 1 bis 3 mils (25,4 µm bis 76,2 µm) der feuchtigkeitsabsorbierende geflockte Holzzellstoffkern (14) von 0,15 Inches (3,8 mm) bis 1,5 Inches (38 mm) dick ist, und die Öffnungen (26) vor dem Kontakt mit Feuchtigkeit von 15% bis 35% des Oberflächenbereichs der Einweg-Barrierelage (22) bilden.

4. Einlage nach Anspruch 3, wobei die Barrierelage (22) mehrere mit Öffnungen versehene Schichten (22a, 22b) hat, wobei die Öffnungen (26) im wesentlichen zueinander ausgerichtet sind, jede Schicht eine im wesentlichen einheitliche Dicke hat und ein Laminat aus einer Basis oder einem Träger und einem Polymer, das sich bei Nässe ausdehnt, aufweist.

5. Einlage nach Anspruch 4, wobei das Polymer ein Stärkepolymer ist.

6. Einlage nach Anspruch 4, wobei das Polymer ein Acrylpolymer ist.

7. Einlage nach Anspruch 3, wobei die Barrierelage (22) eine mit Öffnungen versehene Schicht mit im wesentlichen einheitlicher Dicke ist, die ein oder mehrere Male so gefaltet ist, daß die Öffnungen (26) im wesentlichen zueinander ausgerichtet sind, wobei die Schicht ein Laminat aus einer Basis oder einem Träger und einem Polymer, das sich bei Nässe ausdehnt, aufweist.

8. Einlage nach Anspruch 7, wobei das Polymer ein Stärkepolymer ist.

9. Einlage nach Anspruch 7, wobei das Polymer ein Acrylpolymer ist.

10. Einlage nach einem der vorstehenden Ansprüche, wobei eine Einzellage des absorbierenden Kernmaterials (14) vorhanden ist.

## Revendications

1. Protection destinée à être utilisée par des personnes incontinentes dans un sous-vêtement classique, cette protection comprenant un corps sensiblement en forme de sablier avec des découpes (16, 18) pour laiser passer les jambes de la personne, ledit corps comprenant une couche intérieure (c'est-à-dire située contre la peau de la personne quand la protection est utilisée) (10) de fibres non tissées de polyester ou de rayonne perméables à l'humidité, une couche extérieure (12) de pellicule de polyéthylène imperméable à l'humidité, et une couche centrale (14) de matiére absorbant l'humidité, faite de pâte de bois duvetée, ladite couche intérieure (10) et ladite couche extérieure (12) étant réunies le long du pourtour dudit corps de façon à enfermer ladite couche centrale absorbante (14), une partie (20) située centralement entre lesdites découpes (16, 18) et interposée entre ladite couche intérieure (10) et ladite couche centrale absorbante (14), ladite partie (20) comprenant une couche d'arrêt unidirectionnelle (22) d'épaisseur sensiblement uniforme et une couche buvard (24), perméable à l'humidité, de papier doux, située entre ladite couche d'arrêt (22) et ladite couche intérieure (10), ladite couche d'arrêt (22) comprenant un stratifié contenant un polymére qui gonfle au mouillage, ladite couche d'arrêt (22) étant percée de plusieurs ouvertures (26), lesdites ouvertures (26) ayant un diamètre donnée avant le gonflement dudit polymère pour faciliter le passage du liquide, à travers ladite couche d'arrêt (22), vers ladite couche centrale absorbante (14), le diamètre desdites ouvertures (26) allant en diminuant à mesure que ledit polymère gonfle de manière à restreindre le reflux du liquide, à travers ladite couche d'arrêt (22), vers ladite couche intériéure

perméable (10).

2. Protection selon la revendication 1, comprenant en outre des moyens adhésifs (32, 34) montés sur la couche extérieure (12) pour assujettir de manière amovible ladite protection à un sous-vêtement.

3. Protection selon la revendication 1, dans laquelle ladite couche de fibres non tissées de polyester ou de rayonne perméables à l'humidité pèse de 8,5 à 68 g/m², ladite pellicule de polyéthylène imperméable à l'humidité est épaisse de 12,7 µm à 76,2 µm, ladite couche buvard (24) en papier doux a une épaisseur de 25,4 µm à 76,2 µm, ladite couche centrale (14) de pâte de bois duvetée absorbant l'humidité est épaisse de 3,8 mm à 38 mm, et lesdites ouvertures (26) constituent de 15% à 35% de la superficie de ladite couche d'arrêt unidirectionnelle (22) avant contact avec l'humidité.

4. Protection selon la revendication 3, dans laquelle ladite couche d'arrêt (22) est constituée par plusieurs feuilles (22a, 22b) à ouvertures, lesdites ouvertures (26) étant sensiblement en regard, chaque feuille ayant une épaisseur sensiblement uniforme et comprenant un stratifié d'une base ou d'un porteur et d'un polymère qui gonfle au mouillage.

5. Protection selon la revendication 4, dans laquelle ledit polymère est un polymère d'amidon.

6. Protection selon la revendication 4, dans laquelle ledit polymère est un polymère acrylique.

7. Protection selon la revendication 3, dans laquelle ladite couche d'arrêt (22) est une feuille à ouvertures d'épaisseur sensiblement uniforme repliée une ou plusieurs fois de telle sorte que les ouvertures (26) sont sensiblement en regard, ladite feuille comprenant un stratifié d'une base ou d'un support et d'un polymère qui gonfle au mouillage.

8. Protection selon la revendication 7, dans laquelle ledit polymère est un polymère d'amidon.

9. Protection selon la revendication 7, dans laquelle ledit polymère est un polymère acrylique.

10. Protection selon l'une quelconque des revendications précédentes, dans laquelle il y a une seule couche centrale (14) de matière absorbant l'humidité.

EP 0 139 351 B1

FIG.1

1

EP 0 139 351 B1

F I G.2

F I G.5

2

EP 0 139 351 B1

F I G. 3

F I G. 4

F I G. 6

3